# EUROPEAN PATENT SPECIFICATION

(11) **EP 1 582 151 B1**
(45) Date of publication and mention of the grant of the patent: **02.01.2008**
(21) Application number: 05014934.3
(22) Date of filing: 06.05.1997
(51) Int. Cl.: A61B 17/00

(54) **A device for plugging an opening in a wall of a hollow or tubular organ**
Vorrichtung zum Abdichten einer Öffnung in einem hohlen oder rohrförmigen Organ
Dispositif d'obturation d'un orifice dans une paroi d'organe creux ou tubulaire

(30) Priority: 08.05.1996 SE 9601752
(43) Date of publication of application: 05.10.2005
(62) Divisional of application: 97922269.2
(73) Proprietor: Carag AG, 6340 Baar (CH)
(72) Inventor: Solymar, Laszlo, 427 36 Billdal (SE)
(74) Representative: Bremi, Tobias Hans

(56) References cited:
- EP-A- 0 350 043
- WO-A-96/01591
- DE-A1- 2 822 603
- US-A- 3 874 388

## Description

The present invention relates to a device at an implant for closing as passage as e.g. an aperture through the auricle diaphragm or the ventricle diaphragm of a heart or in a body channel, and comprising a closing body which is fixable at the passage.

Such a device is known from WO 96/01591.

By means of EP, B1, 0 362 113 it is previously known a device for closing a passage in the heart of patients, wherein the closing part run the risk of tipping over and thereby causing the passage to be exposed for passing through the heart. The cause of the insecure closing capacity is the insecure manoeuvrability for the closing part when applied around the passage and that the application takes part long before it has arrived on its final position around the passage in the heart.

The main object of the present invention is therefore primarily to try to solve the described problem with manoeuvrability and application.

Said object is achieved by means of a device according to claim 1, which is substantially characterized in that a fluid tight closing body which is expanding and stiffening in a radial direction is arranged to be built up at the position of the intended closing spot, after insertion through a body vein.

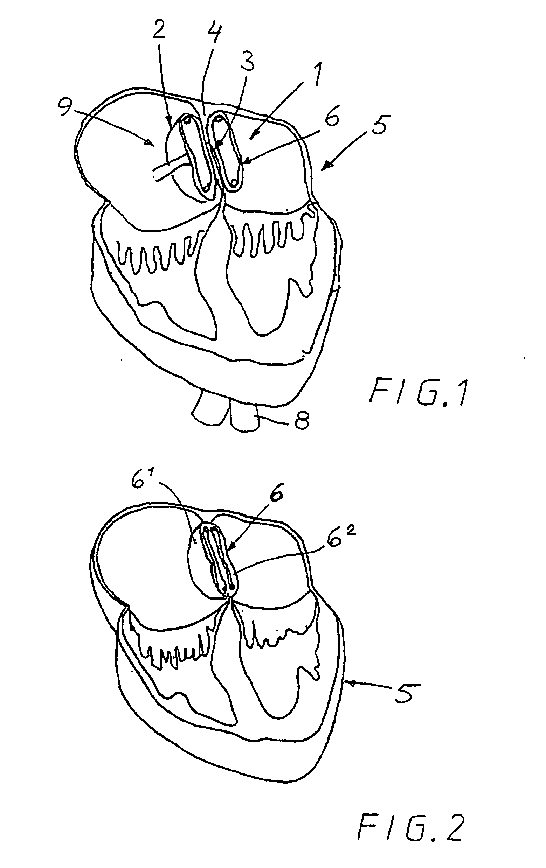

The invention will be described here below as an number of preferred embodiments, wherein reference is made to the accompanying drawings, in which Fig. 1 shows a section through a heart during application of the implant in it.
Fig 2 shows the implant positioned in the heart,
Fig 3-5 an implant wherein,

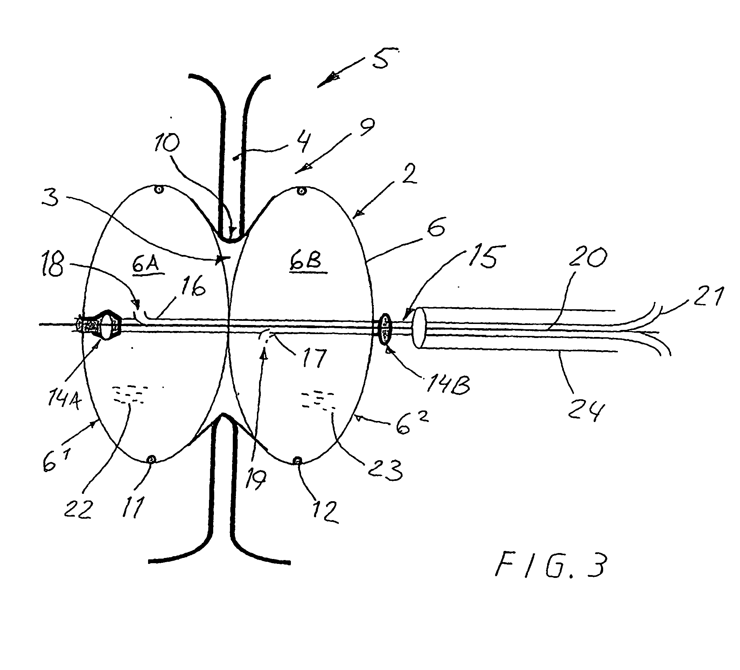

Fig 3 shows the implant moved into position before fixing,

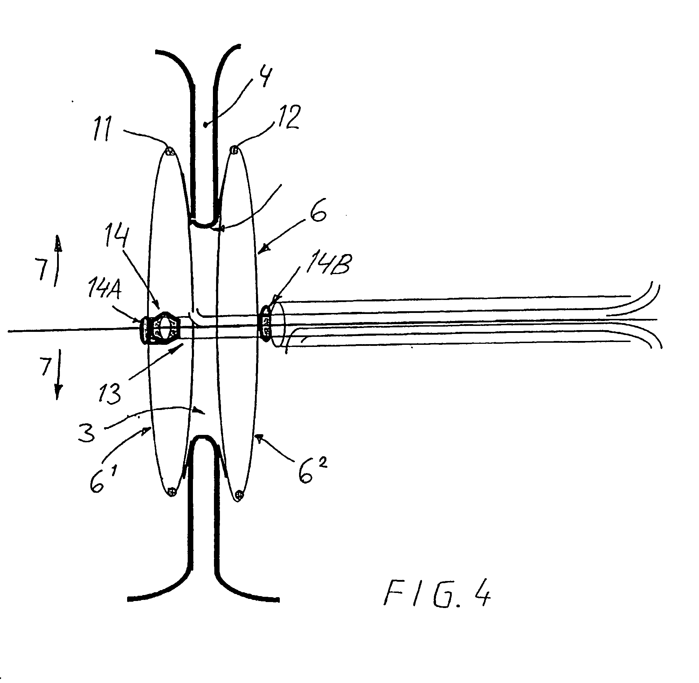

Fig 4 shows the implant below a fixing position,

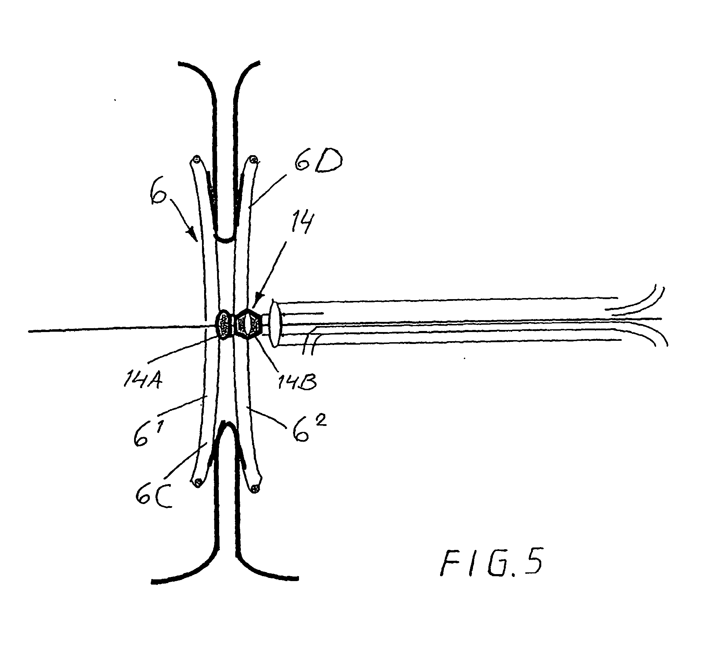

Fig 5 shows the implant in its fixing position,

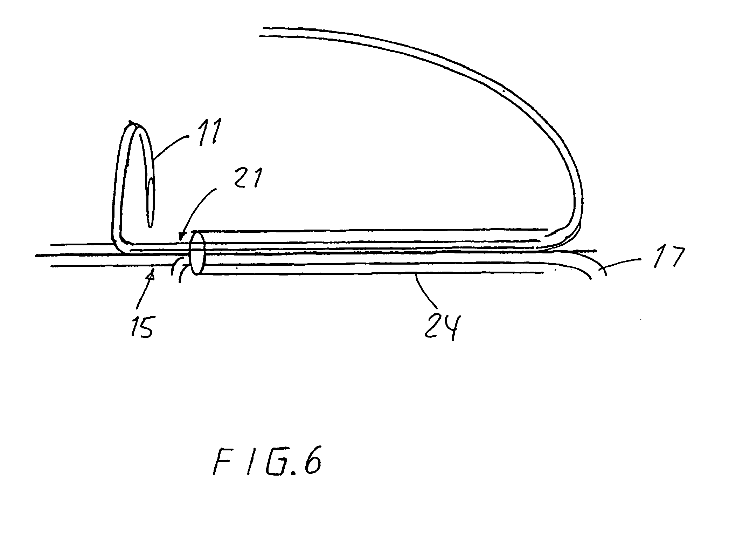

Fig 6 shows application of a spiral thread via sheaths, and

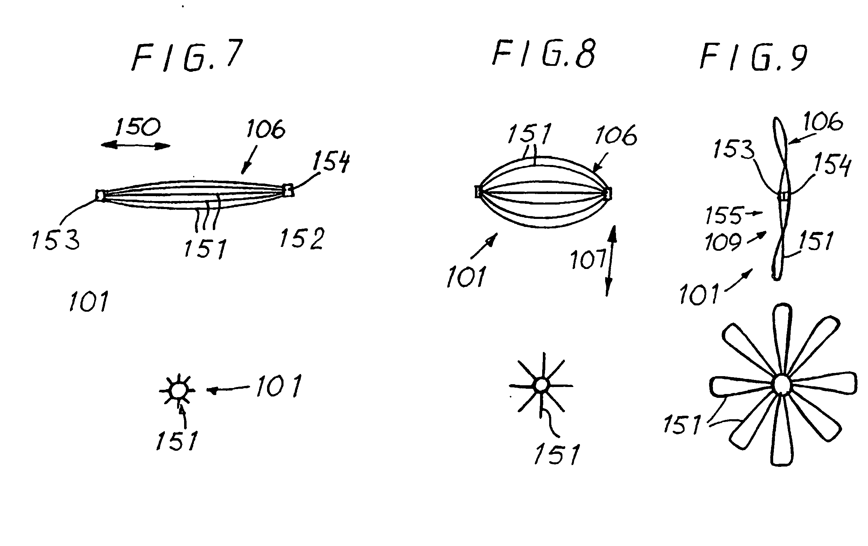

Fig 7-9 shows an implant according to the invention, wherein
Fig 7 shows an initial position for said implant seen from the side and from one end respectively,

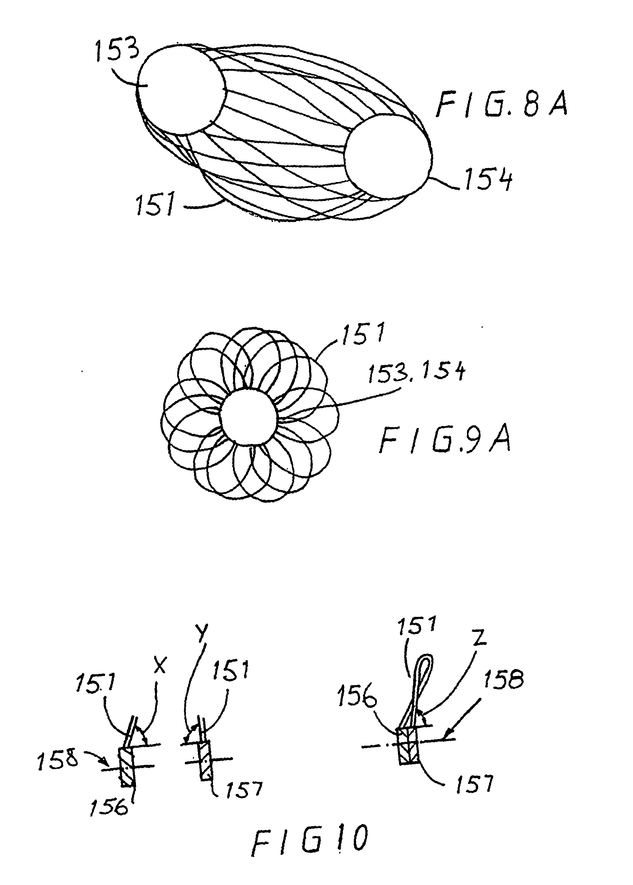

Fig 8 shows said implant at a between position during extension seen from the side and one end respectively,
Fig 9 shows the implant completely extended seen from the side and one end respectively,
Fig 8A shows the implant in a perspective view during extension,
Fig 9A shows an end view of the implant in its extended position, and
Fig 10 shows schematically the principle for building up the hub and connection of the implant to it and the final position of the implant in completely extended condition seen from the side.

The invention relates to a preferably cardiological implant by means of which it is possible e.g. to close an aperture through the auricle diaphragm or the ventricle diaphragm of a heart.

The implant according to the present invention is arranged to be built up at the location in the body e.g. the heart, in contrast to known implant e.g. so called umbrellas and sails that are instead extended as soon as the compressed umbrella leaves its insertion sheath.

A device 1 at an implant 2 for closing an internal passage 3, like e.g. an aperture in the auricle diaphragm 4 or the ventricle diaphragm of a heart 5 or in a desired body channel which one wishes to close concerns a closing body 6 which is to be built up at the location. More exactly, a device which is shown on the drawings in Fig 1-5, is formed by a in radial direction 7 expanding and stiffening fluid tight closing body 6. This body 6 is arranged to be built up at the position 9 of the intended closing spot, after insertion through a body vein 8.

Said closing body 6 consists of an inflatable balloon, preferably double balloon of thin and non-thrombogenic material. A connection part 10 between the two balloon elements 6A, 6B is arranged to form a guidance for the two balloon elements 6A, 6B around the peripheral edge 3A of said aperture 3.

Both chambers 6A, 6B of the balloon 6 are arranged to be dilated radially 7 by means of a number of stiffening means 11, 12 which in the first shown example is realised in the shape of a coil spring which in each case is received in the respective balloon chamber 6¹, 6², in order to dilate said balloon chamber 6¹, 6² radially in the operational position and thereby bring about an efficient holding of the balloon around the aperture 3, as is shown in Fig 4.

A centrally, at the middle 13 of the balloon located locking mechanism 14 is arranged mutually interconnect both balloon chambers 6¹, 6² at their central part. Suitably, the aforementioned locking mechanism 14 is divided into two with each respective locking member 14A, 14B attachable to the outer wall 6C, 6D of the respective balloon chamber 6¹, 6². For example, the aforementioned locking members 14A, 14B may be of a prior art type, e.g. as snap together members.

The described implant 2 is delivered to the location for application in the form of a tightly over an inner sheath 15 rolled up double balloon 6 of thin, durable and physically friendly non-thrombogenic material. Two narrow delivery catheters 16, 17 extend through said inner sheath 15 of which the distal delivery catheter 16 has its outlet opening 18 in the distal balloon 6¹ and in a corresponding manner, the proximal catheter 17 has its outlet opening 19 in the proximal balloon 6²:

By means of the well known Seldinger technique, a vein inserter of dimension 11 F is inserted into the femoral vein. A catheter is placed in the left upper pleural vein and through this is passed a conductor 20 which is left behind for subsequent implant work. At the same time, this constitutes a part of a safety system against unintentional release, because the conductor 20 locks the fastening mechanism between the inner sheath 15 and the implant 2. Over the conductor 20, the implant 2 is introduced into the vein inserter, then in the lower vena cava and further up to the heart 5 until the central mark reaches the middle of the aperture 3 or any other defect which is desired to be closed. After that, the distal balloon 6¹ is filled with contrast fluid via the distal delivery catheter 16, whereupon the intended metal spiral 11 is inserted into the delivery catheter 21 and is fed in until it rolls itself up inside the distal balloon 6¹. In a corresponding way, the proximal balloon 6² is filled with contrast fluid in order to enable the parts to be visible via roentgen inside the body during the work to move them into position. After that a metal spiral 12 is also delivered to the proximal balloon 6², as is shown in Fig 3. So when the metal spirals 11, 12 or other as stiffeners functioning means are located in the correct position, they are released by backing the constraining mandrins out of the metal spirals 11, 12. The contrast fluid 22, 23 is evacuated out from the balloons 6¹, 6² and the distal part 14 of the locking mechanism is pulled through its proximal ring 14B in the locking position. The conductor 20 is then pulled out accompanied by the inner sheath 15 which now runs freely and is pulled out from the distal balloon attachment and also the outer enclosing delivery sheath 24.

The implant according to the invention, which is shown in Fig 7-10 departs from the above described device, foremost by instead of using metal spirals as dispersing force which maintains a balloon or other occluding sail in position, utilising the rotating quality of non-bendable metals when they are bent, and when the ends move past a critical point. For this object, there are thin nitinol treads attached around an inner core for example in each end for example inside a balloon according to the above. The distal and the proximal balloons has a further opening between them and the diameter of the balloon at its smallest part corresponding to the ASD-size, i.e. that it is somewhat but not much larger. The balloons are inflated by means of a contrast fluid whereupon the furthermost, as seen in the direction of insertion, attachment point of the nitinol threads are slowly pulled back at the same time as the nearest, as seen in the direction of insertion, are pushed ahead, i.e. in the direction toward each other. At a critical position, the threads are twisted sideways and maintains a circular shape, which desired shape is used according to the present invention. In position, the balloons are evacuated and the threads are locked in the twisted spring-like position.

More precisely, with reference to said figures 7-10 are formed a device 101 at an implant for closing a passage, for example according to the above described embodiment, and which comprises a closing body 106 which is attachable at said passage, alone or in combination with further sealing means in a radial direction 107 expanding stiffening fluid tight closing body. Said body 106 is arranged that after insertion through a not shown body vein in its longitudinal direction 150, to be built up at the position 109 for the intended closing spot.

A number of threads 151 which in the shown embodiment are eight in number are arranged of such a material and with such qualities that they twist automatically sideways to form a circular shape, as is shown in Fig 9 and are locked in the twisted coil-like position for interconnecting the two balloon chambers etc. against each other. The body 106 is preferably formed by a plurality of thin nitinol threads which are attached to a respective in axial direction 150, 152 relative to each other movable holder nuclei 153, 154. The threads 151 are arranged to twist sideways in the same direction and then to assume a circular shape 155, similar to a flower, a propeller or an umbrella with circular shape and arranged with suitable means, for closing said passage, e.g. that layers of watertight material are connected to the threads 151.

In the example according to Fig 10 is shown how the threads 151 are attached with different inclination x, y with reference to the respective separate holder nuclei 156, 157. For this object, the threads 151 are arranged to be mutually forced to be directed toward that holder nucleus 157 from which the threads 151 depart with the largest angle y, as calculated from the centre axis 158 of the holder nuclei, with similar angle z for the different threads 151.

The invention has been carefully described in the above mentioned example and therefore the idea should be clearly understood and the invention is neither limited to the above described and on the drawings shown embodiment, but may be varied within the scope of the claims without departing from the concept of the invention.

## Claims

1. An implant to close an aperture in a location of a body, as e.g. an aperture through the auricle diaphragm or the ventricle diaphragm of a heart or in a body channel, the implant comprising
a fluid tight closing body (106) which is arranged to be built up and fixable at a position of an intended closing spot within the aperture after insertion through a body vein and
the implant further comprising a number of thin elastic threads (151) each comprising a first and a second end, the elastic threads being capable of maintaining the closing body (106) in its expanded position,
and a first and a second holder nucleus (153, 154; 156, 157) being movable relative to each other in an axial direction,
wherein the elastic threads (151) are attached with their first and second ends to the first and second holder nucleus (153, 154; 156, 157) respectively such that the threads (151) are bent and assume a circular shape when the first and second holder nucleus (153, 154; 156, 157) are moved relative to each other, wherein the closing body (106) is expanding and stiffening in a radial direction,
**characterized in that**
the threads (151) are lockable in a position where the first and second ends have moved relative to each other past a critical point, wherein this critical point is defined by the rotating quality of non-bendable metals, and wherein the threads (151) are arranged to twist sideways in the same direction when passing this critical point and to assume a circular shape and wherein the multiple of threads (151) maintains a circular shape when the threads (151) have passed this critical point, and **in that** layers of occluding, in particular watertight material are connected to the threads (151).

2. A device according to claim 1, **characterized in that** the closing body (6) consists of an inflatable balloon of thin and non-thrombogenic material.

3. A device according to claim 2, wherein the closing body (6) is formed by a cardiological implant (2) for closing an aperture (3) in a diaphragm wall (4) in a heart (5) , **characterized in that** the balloon (6) is formed by a double balloon at which a connection part (10) between the two balloon elements (6A, 6B) is arranged to form a guidance for the two balloon elements (6A, 6B) around the circumference edge (3A) of said aperture (3).

4. A device according to claim 3, **characterized in that** both chambers (6¹, 6²) of the balloon (6) are arranged to be dilated radially (7) by means of a stiffening means (11, 12 ) .

5. A device according to claim 4, **characterized in that** a number of threads (151) are arranged to twist sideways to form a circular shape and be locked in the twisted spring like position interconnecting the two balloon chambers against each other.

6. The implant according to claim 1, **characterized in that** the threads (151) are attached to the holder nuclei (153, 154; 156, 157) at an angle, calculated from a centre axis (158) of each holder nucleus (153, 154; 156, 157).

7. The implant according to one of claims 1 to 6, wherein the threads (151) are nitinol threads.

## Patentansprüche

1. Implantat zum Verschliessen einer Öffnung an einer Stelle eines Körpers, wie zum Beispiel eine Öffnung durch das Aurikeldiaphragma oder das Ventrikeldiaphragma eines Herzes oder oder in einem Körperkanal, wobei das Implantat
einen flüssigkeitsdichten Verschlusskörper (106) aufweist, welcher angeordnet ist aufgebaut zu werden und fixierbar zu sein in einer Position eines gewünschten Verschlusspunktes innerhalb der Öffnung nach Einführung durch eine Körpervene, und
wobei das Implantat weiter eine Zahl von dünnen elastischen Fäden (151) aufweist, jeder umfassend ein erstes und zweites Ende, wobei die elastischen Fäden dazu in der Lage sind, den Verschlusskörper (106) in seiner expandierten Lage zu halten,
und einen ersten und zweiten Haltekern (153, 154; 156, 157), welche relativ zueinander in axialer Richtung beweglich sind,
wobei die elastischen Fäden (151) mit ihrem ersten respektive zweiten Ende mit dem ersten respektive zweiten Haltekern (153, 154; 156, 157) verbunden sind, so dass die Fäden (151) gebogen sind und eine Kreisform annehmen, wenn der erste und zweite Haltekern (153, 154; 156, 157) relativ zueinander bewegt werden, wobei der Verschlusskörper (106) in einer radialen Richtung expandiert und versteift
**dadurch gekennzeichnet, dass**
die Fäden (151) einrastbar sind in einer Position, bei welcher die ersten und zweiten Enden relativ zueinander über einen kritischen Punkt hinaus bewegt worden sind, wobei dieser kritische Punkt definiert ist durch die Rotationsqualität des nicht-biegbaren Metalls, und wobei die Fäden (151) angeordnet sind, um sich seitlich in der gleichen Richtung zu verdrehen, wenn dieser kritische Punkt überschritten wird und eine Kreisform anzunehmen, und wobei die Mehrzahl von Fäden (151) eine Kreisform bewahrt, wenn die Fäden (151) diesen kritischen Punkt überschritten haben und dass Lagen von verschliessendem, insbesondere wasserdichtem Material mit den Fäden (151) verbunden sind.

2. Eine Vorrichtung gemäss Anspruch 1, **dadurch gekennzeichnet, dass** der Verschlusskörper (6) aus einem aufblasbaren Ballon aus dünnen und nicht-thrombogenem Material besteht.

3. Eine Vorrichtung gemäss Anspruch 2, wobei der Verschlusskörper (6) aus einem kardiologischen Implantat (2) zur Verschliessung der Öffnung (3) in einer Diaphragmawand (4) eines Herzens (5) gebildet ist, **dadurch gekennzeichnet, dass** der Ballon (6) aus einem doppelten Ballon gebildet ist, an welchem ein Anschlussteil (10) zwischen den zwei Ballonelementen (6A, 6B) angeordnet ist um eine Führung für die zwei Ballonelemente (6A, 6B) und den Umfang um die Umfangskante (3A) der genannten Öffnung (3) zu bilden.

4. Eine Vorrichtung gemäss Anspruch 3, **dadurch gekennzeichnet, dass** beide Kammern (6¹, 6²) des Ballons (6) angeordnet sind um radial aufgeweitet (7) zu werden mittels Versteifungsmitteln (11, 12).

5. Eine Vorrichtung gemäss Anspruch 4, **dadurch gekennzeichnet, dass** eine Anzahl Fäden (51) angeordnet sind um sich seitlich zu verdrehen um eine Kreisform anzunehmen und um in der verdrehten federähnlichen Position verschlossen zu werden, wobei sie in dieser Position die zwei Ballonkammern gegenseitig verbinden.

6. Das Implantat gemäss Anspruch 1, **dadurch gekennzeichnet, dass** die Fäden (151) an den Halterungskernen (153, 154; 156, 157) in einem Winkel angeordnet sind, gerechnet von der Mittelachse (158) eines jeden Halterungskerns (153, 154; 156, 157).

7. Das Implantat gemäss einem der Ansprüche 1-6, wobei die Fäden (151) Nitinolfäden sind.

## Revendications

1. Implant destiné à fermer une ouverture située dans un corps, par exemple une ouverture traversant la cloison inter-auriculaire ou la cloison inter-ventriculaire du coeur ou dans un canal corporel, l'implant comprenant :
un organe d'obturation étanche aux fluides (106) prévu pour être monté et fixé en une position de fermeture souhaitée à l'intérieur de l'ouverture après son insertion à travers une veine et
l'implant comprenant en outre un certain nombre de minces fils élastiques (151) comprenant chacun une première et une deuxième extrémités, les fils élastiques étant capables de maintenir l'organe d'obturation (106) dans sa position déployée,
et un premier et un deuxième noyaux de support (153, 154 ; 156, 157) déplaçables l'un par rapport à l'autre dans une direction axiale,
les fils élastiques (151) étant attachés par leurs premières et deuxièmes extrémités au premier et au deuxième noyaux de support (153, 154 ; 156, 157) respectivement, de telle sorte que les fils (151) soient fléchis et adoptent une forme circulaire lorsque le premier et le deuxième noyaux de support (153, 154 ; 156, 157) sont déplacés l'un par rapport à l'autre,
l'organe d'obturation (106) étant déployé et rigidifié dans une direction radiale,
**caractérisé en ce que**
les fils (151) peuvent être bloqués dans une position dans laquelle les première et deuxième extrémités se sont déplacées l'une par rapport à l'autre au-delà d'un point critique, ce point critique étant défini par la capacité de torsion de métaux non flexibles, et les fils (151) étant agencés de manière à se tordre latéralement dans la même direction en dépassant ce point critique et à adopter une forme circulaire, et la pluralité de fils (151) conservant une forme circulaire lorsque les fils (151) ont dépassé ce point critique, et **en ce que** des couches de matériau d'obturation, en particulier étanche à l'eau, sont connectées aux fils (151).

2. Dispositif selon la revendication 1, **caractérisé en ce que** l'organe d'obturation (6) se compose d'un ballon gonflable en matériau mince et non thrombogène.

3. Dispositif selon la revendication 2, dans lequel l'organe d'obturation (6) est formé par un implant cardiologique (2) pour fermer une ouverture (3) dans une cloison (4) du coeur (5), **caractérisé en ce que** le ballon (6) est formé par un double ballon au niveau duquel est agencée une partie de connexion (10) entre les deux éléments de ballon (6A, 6B) pour former un guide pour les deux éléments de ballon (6A, 6B) autour du bord circonférentiel (3A) de ladite ouverture (3).

4. Dispositif selon la revendication 3, **caractérisé en ce que** les deux chambres (6¹, 6² ) du ballon (6) sont agencées de manière à se dilater radialement (7) par le biais d'un moyen de rigidification (11, 12).

5. Dispositif selon la revendication 4, **caractérisé en ce qu'**un certain nombre de fils (151) sont agencés de manière à se tordre latéralement pour former une forme circulaire et pour être bloqués dans la position tordue de type ressort en reliant entre elles les deux chambres du ballon l'une contre l'autre.

6. Implant selon la revendication 1, **caractérisé en ce que** les fils (151) sont attachés aux noyaux de support (153, 154 ; 156, 157) suivant un certain angle, calculé à partir d'un axe médian (158) de chaque noyau de support (153, 154 ; 156, 157).

7. Implant selon l'une quelconque des revendications 1 à 6, dans lequel les fils (151) sont des fils de nitrinol.
